# EUROPEAN PATENT APPLICATION

(11) **EP 3 138 552 A1**
(43) Date of publication of application: **08.03.2017**
(21) Application number: 15785484.5
(22) Date of filing: 27.04.2015
(51) Int. Cl.: A61K 8/26, A61K 8/02, A61Q 1/02

(54) **COSMETIC**

(30) Priority: 28.04.2014 JP 2014092373
(71) Applicant: Sakai Chemical Industry Co., Ltd., Sakai-shi, Osaka 590-8502 (JP); Rohto Pharmaceutical Co., Ltd., Osaka-shi Osaka 544-8666 (JP)
(72) Inventor: OTA, Mayu, Sakai-shi Osaka 590-0985 (JP); TSUJITA, Hiroshi, Sakai-shi Osaka 590-0985 (JP); MORI, Kenji, Sakai-shi Osaka 590-0985 (JP); KOBAYASHI, Keita, Sakai-shi Osaka 590-0985 (JP)
(74) Representative: Isarpatent
(86) International application number: PCT/JP2015/062645
(87) International publication number: WO 2015/166895

(57) **Abstract**

The inventors have investigated a fluorescent substance being safe and having a high color developing property, and it is one of objects of the present disclosure to obtain an inorganic red fluorescent substance consisting of an element which exerts no bad influence upon human bodies.

A cosmetic containing inorganic particles consisting of a compound represented by a general formula:

CaₓAl_{y}O_{(2x+3y+4z)/2}:Mn⁴⁺_{z}

wherein 0.1<x<1.05, 11.9<y≦12, and 0.0005<z<0.1.

## Description

### TECHNICAL FIELD

The present disclosure relates to a cosmetic containing an aluminate composite oxide.

### BACKGROUND OF THE DISCLOSURE

It is known that a fluorescent substance is mixed in a cosmetic (for example, patent document 1). Such cosmetic is expected that the color of skin is adjusted by color development derived from the fluorescent substance and an unconventional cosmetic property is shown by specific color derived from the fluorescence. Especially, a compound having a red fluorescence is expected to produce an effect of making the facial color appear bright by providing reddish tint to the skin.

However, an organic fluorescent substance such as 3-hydroxypyrene-5, 8, 10-trisulfonic acid which has been proposed, and an inorganic fluorescent substance such as NaCl:Mn have not been used substantially because their safety is not confirmed. On the other hand, mineral-based fluorescent substances such as axinite (aluminum calcium borosilicate), calcite (calcium carbonate), and petalite (aluminum lithium silicate) that are considered relatively safe have been proposed (see patent document 2) but they may not be used in a cosmetic because of an insufficient color development.

A compound represented by a general formula CaAl₁₂O₁₉:Mn⁴⁺ has been known and disclosed in nonpatent document 1. However, the compound has been investigated as a fluorescent substance for LED and has not been known as a cosmetic component.

### PRIOR TECHNICAL DOCUMENTS

### PATENT DOCUMENTS

[Patent Document 1] Japanese Kokai Publication Hei5-117127
[Patent Document 2] Japanese Kohyo Publication 2003-500429

### NONPATENT DOCUMENT

[Nonpatent Document 1] A. Bergstein et al, "Manganese-Activated Luminescence in SrAl12O19 and CaAl12O19" J. Electrochem. Soc., 118, p.1166 (1971)

### SUMMARY OF INVENTION

### PROBLEMS TO BE SOLVED BY THE INVENTION

The inventors have investigated a fluorescent substance being safe and having a high color developing property, and further have investigated to obtain an inorganic red fluorescent substance consisting of an element which exerts no bad influence upon human bodies.

### MEANS FOR SOLVING OBJECT

The present disclosure relates to a cosmetic containing inorganic particles consisting of a compound represented by a general formula:

CaₓAl_{y}O_{(2x+3y+4z)/2}:Mn⁴⁺_{z}

wherein 0.1<x<1.05, 11.9<y≦12, and 0.0005<z<0.1.

The inorganic particle content is preferably 0.1 to 100 wt% relative to the total amount of the cosmetic.

The inorganic particles preferably have a hexagonal plate shape.

The inorganic particles preferably have an average particle diameter of 5 to 100 µm.

The inorganic particles preferably have a luminance Y of 0.1 or more when a light having an excitation light wavelength of 450 nm is irradiated.

### EFFECTS OF THE INVENTION

The cosmetic of the present disclosure has an excellent color developing property and a high safety.

### BRIEF DESCRIPTION OF THE DRAWINGS

FIG. 1 is a SEM photograph of particles obtained in Example 1 (magnification: 1000 magnifications).
FIG. 2 is a SEM photograph of particles obtained in Example 1 (magnification: 2000 magnifications).
FIG. 3 is a SEM photograph of particles obtained in Example 2 (magnification: 1000 magnifications).
FIG. 4 is a SEM photograph of particles obtained in Example 2 (magnification: 2000 magnifications).
FIG. 5 is a SEM photograph of particles obtained in Example 3 (magnification: 1000 magnifications).
FIG. 6 is a SEM photograph of particles obtained in Example 3 (magnification: 2000 magnifications).
FIG. 7 is a SEM photograph of particles obtained in Example 4 (magnification: 1000 magnifications).
FIG. 8 is a SEM photograph of particles obtained in Example 4 (magnification: 2000 magnifications).
FIG. 9 is a SEM photograph of particles obtained in Example 5 (magnification: 1000 magnifications).
FIG. 10 is a SEM photograph of particles obtained in Example 5 (magnification: 2000 magnifications).
FIG. 11 is a SEM photograph of particles obtained in Example 6 (magnification: 500 magnifications).
FIG. 12 is a SEM photograph of particles obtained in Example 7 (magnification: 2000 magnifications).

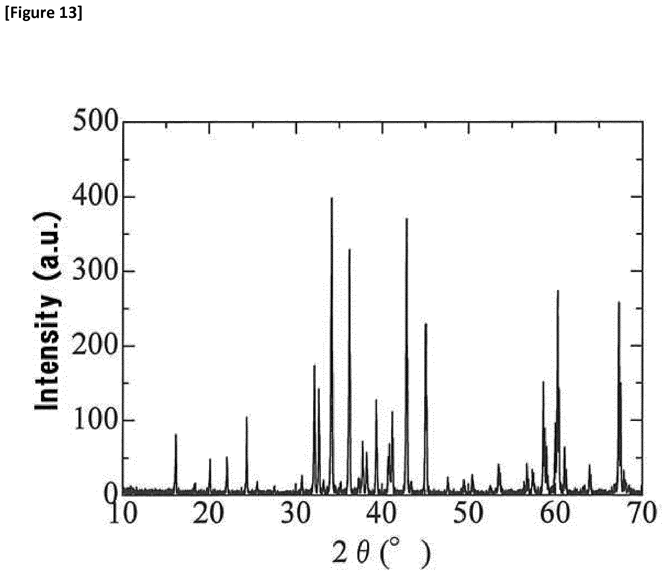

FIG. 13 is a XRD pattern of particles obtained in Example 1.

### DETAILED DESCRIPTION OF PREFERRED EMBODIMENTS

The present disclosure relates to a cosmetic containing inorganic particles consisting of a compound represented by a general formula: CaₓAl_{y}O_{(2x+3y+4z)/2}:Mn⁴⁺_{z} (in the formula, 0.1<x<1.05, 11.9<y≦12, and 0.0005<z<0.1). That is, the cosmetic contains inorganic particles consisting of a compound obtained by doping a compound represented by a general formula CaₓAl_{y}O_{(2x+3y)/2} with Mn⁴⁺. CaAl₁₂O₁₉:Mn⁴⁺ red fluorescent substance had been developed in 1971 (nonpatent document 1). In nonpatent document 1, it is disclosed that Mn⁴⁺ generates red light in the wavelength near 657 nm by removing an octahedron site of CaAl₁₂O₁₉.

Recently, research and development of CaAl₁₂O₁₉:Mn⁴⁺ as red fluorescent substance for white LED have been carried out. However, such red fluorescent compound has not been examined as a material for a cosmetic.

The inventors found that the inorganic particle of the present disclosure may be used suitably as a material for a cosmetic. That is, the inorganic particle of the present disclosure is safe material which is harmless to human health, and has a high stability and an excellent red color developing property when used. Therefore, a cosmetic showing an unprecedented and good cosmetic appearance can be obtained.

The inorganic particle of the present disclosure satisfies the requirement that x, y, and z in the formula are within the above-mentioned range. The x, y, and z are preferably set within the following range because a suitable fluorescent properties may be achieved. The x is more preferably more than 0.9 and less than 1.0. The z is more preferably more than 0.001 and less than 0.05.

The inorganic particles of the present disclosure preferably have a luminance Y in xyz representation system (hereafter described as a luminance Y) of 0.1 or more when a light having an excitation light wavelength of 450nm is irradiated. When the inorganic particle having a luminance of less than 0.1 are used in a cosmetic, the reddish tint is hardly recognized. The luminance depends on the composition and additives mentioned later, so that the luminance Y may be set at 0.1 or more by adjusting the above-mentioned elements. The luminance Y is preferably 0.1 or more, and more preferably 0.5 or more. In this specification, the luminance Y is measured by the method disclosed in the example.

The inorganic particle of the present disclosure may contains other metals such as alkaline metals including Li, Na, and K, alkaline earth metals including Be, Mg, Sr, Ba, and Ra, and other metals including Ti, Zr, V, Cr, Mo, W, Fe, Co, Ni, Pd, Pt, Cu, Ag, Zn, Al, Ga, Si, Ge, and Sn within such a range as not to affect safety for a human body and performances. The defined content is not particularly limited but the content of the other metal is preferably 10 wt% or less relative to the inorganic particle of the present disclosure.

The shape and particle diameter of the inorganic particle of the present disclosure are not particularly limited and an inorganic particle having a hexagonal plate shape may be used. The hexagonal plate-shaped particles have excellent performances in the smoothness, adhesive property and soft focus property so that the particles can be preferably used as a material for a cosmetic.

It is desirable that the particle diameter of the hexagonal plate-shaped inorganic particles of the present disclosure is 5 to 100 µm. When the particle diameter is less than 5 µm, the emission intensity may be reduced. If the particle diameter is more than 100 µm, the roughness may be generated when the particles are used in a cosmetic. The particle diameter is D50 measured by using a laser diffraction particle size distribution analyzer.

A ratio between a long diameter and a thickness of the hexagonal-plated inorganic particles of the present disclosure is desirably 0.1 to 1. More desirably, the ratio is 0.1 to 0.5. When the ratio is less than 0.1, the smoothness, the adhesive property and the soft focus property may be deteriorated. When the ratio is more than 1, the emission intensity may be reduced. The long diameter and the thickness are average values of values determined by measuring 250 particles in an image of 1000 magnification taken by using a scanning electron microscope (JSM 840F manufactured by JEOL Ltd.).

The inorganic particles of the present disclosure preferably have a BET specific surface area (it is also called as specific surface area) of 0.03 to 0.8 m²/g. When the BET specific surface area is less than 0.03 m²/g, a concealing property is low so that toning may become difficult. When the BET specific surface area is more than 0.8 m²/g, coagulated particles may be generated to reduce the dispersibility. The upper limit is more preferably 0.5 m²/g.

A method for producing the inorganic particles of the present disclosure is not particularly limited but the particles may be obtained by a method comprising a step of mixing a Ca source compound, a Mn source compound, and an Al source compound as the raw material in accordance with the mole ratio of the intended compound to prepare a precursor and a step of calcinating.

The Ca source compound may include calcium carbonate, calcium hydroxide, calcium oxide, calcium chloride, calcium nitrate, calcium sulfate, calcium fluoride, calcium bromide, and calcium iodide. Two or more of these compounds may be used in combination.

The Al source compound may include aluminum oxide, aluminum chloride, and aluminum sulfate. Two or more of these compounds may be used in combination.

The Mn source compound may include manganese carbonate, manganese acetate, manganese oxide, manganese sulfate, manganese nitrate, manganese fluoride, and manganese bromide. Two or more of these compounds may be used in combination.

A flux component may be used according to need at the time of calcinating. The flux component promotes the particle growth or suppress the growth of specified face to make it easy to generate hexagonal plate-shaped particles. Further, it is preferred that the crystallinity becomes high and the emission intensity is improved.

The flux component is not particularly limited but may include calcium fluoride, magnesium fluoride, aluminum fluoride, ammonium fluoride, magnesium chloride, calcium chloride, ammonium chloride, magnesium bromide, calcium bromide, ammonium bromide, magnesium iodide, calcium iodide, ammonium iodide, boric acid, boron oxide, sodium metaborate, and sodium tetraborate. Two or more of these compounds may be used in combination.

An amount of the flux component to be used is preferably 0 to 20 mol% relative to the inorganic particles of the present disclosure. When the amount is more than the disclosed value, the emission intensity may be reduced.

A method for mixing these material compounds may be any known method. For example, a method which comprises preparing a water-based dispersion of the material compound and mixing while stirring or pulverizing by using a wet media mill and then evaporative drying the whole quantity, or a method which comprises dry mixing using a general mixing apparatus such as a henschel mixer, and a tumbler, or a hammer mill, a high pressure air jet mill, or a combination thereof may be used.

A calcinating method may be any known method. For example, a method of calcinating with a ceramics crucible or a method of calcinating with a rotary kiln while rotating may be used.

The calcinating is preferably performed under oxidizing calcinating condition such as in the atmosphere. A calcinating temperature is not particularly limited but preferably 1000 to 1800°C, more preferably 1300 to 1600°C. When the temperature is less than 1000°C, the emission intensity may be reduced. When the temperature is more than 1800°C, the particles may be strongly calcinated to deteriorate the dispersibility.

The particles after calcinating may be subjected to post-treatments such as filtration through a sieve, water washing and other washing, and pulverization as necessary. The particles obtained by repeating once or more times the steps of washing with an acid or alkali after calcinating and calcinating again are especially preferred because they have an improved emission intensity.

The inorganic particle of the present disclosure may be blended in a cosmetic as it is but may be subjected to a known surface treatment according to need before blending.

The surface treatment is not particularly limited but any material may be used for the surface treatment if the material can be used for a cosmetic. A coating layer composed of inorganic compounds such as oxides, hydroxides, carbonates, and phosphates of silicone, zinc, titanium, aluminum, zirconium, and tin may be provided. Further, for giving water repellency, dimethylpolysiloxane, methylhydrogenpolysiloxane, methylphenylpolysiloxane, methylmethoxypolysiloxane, dimethylpolysiloxane dihydrogen, and copolymers thereof, stearic acid, lauric acid, oleic acid, and metal salts thereof (aluminum salt, zinc salt, magnesium salt, and calcium salt), polyvinyl alcohol, ethylene glycol, propylene glycol, monoethanolamine, aminomethylpropanol, diethanolamine, triethanolamine, monopropanolamine, dipropanolamine, tripropanolamine, paraffin wax, polyethylene wax, aminosilane, epoxysilane, methacrylsilane, vinylsilane, mercaptosilane, chloroalkylsilane, alkylsilane, fluoroalkylsilane, hexamethylsilazane, hexamethylcyclotrisilazane, trimethylolpropane, trimethylolethane, and pentaerythritol may be included.

The surface treatment may be performed using a single compound, a lamination process or a mixing process may be performed by using two or more compounds in combination. Further, a coating layer of an organic compound may be provided after the treatment of the inorganic compound, but it is important not to reduce the original lubricating property.

The coating amount of the inorganic compound and the organic compound is preferably 0.1 to 30 wt% relative to the inorganic particles, more preferably 0.1 to 20 wt%. When the coating amount is 0.1 wt% or more, the function improving effect by the surface treatment can be exerted. When the coating amount is 30 wt% or less, the treatment may be performed without damaging the original lubricating property, and it is advantageous from economic viewpoint.

A method of the surface treatment is not particularly limited but may be performed by a method comprising adding the inorganic compound or the organic compound to a water-based dispersion of the inorganic particles and then adjusting the pH of the dispersion to coat. Further, water-insoluble organic compounds may be surface-treated by dry mixing an organic compound, and pulverizing or mixing followed by heating according to need for coating the water-insoluble organic compound.

The cosmetic of the present disclosure preferably contains the inorganic particles of the present disclosure of 0.1 to 100 wt%, more preferably 0.1 to 50 wt%. When the content is less than 0.1 wt%, it is not preferred because the above-mentioned effect cannot be obtained sufficiently. When the content is over 50 wt%, it is not preferred because the content of the powder of the present disclosure is excess so that the degree of freedom for cosmetic composition is reduced to make it difficult to handle.

Examples of the cosmetic of the present disclosure may include a foundation, a makeup base, an eye shadow, a rouge, a mascara, a lipstick and a sunscreen agent. The cosmetic of the present disclosure can be in any form such as that of an oil-based cosmetic, a water-based cosmetic, an O/W type cosmetic or a W/O type cosmetic. Above all, the cosmetic of the present disclosure can be suitably used in makeup cosmetics including a foundation, a makeup base, and an eye shadow and sunscreen agents.

For the cosmetic of the present disclosure, any aqueous component or oily component that can be used in the field of cosmetics can be used in combination in addition to the inorganic particle of the present disclosure. The aqueous component and oily component described above are not particularly limited, and examples thereof may include those containing components such as oils, surfactants, moisturizers, higher alcohols, sequestrants, natural and synthetic polymers, water-soluble and oil-soluble polymers, UV blocking agents, various extracts, inorganic and organic pigments, inorganic and organic clay minerals and other various powders, inorganic and organic pigments treated with metallic soap or silicone, coloring materials such as organic dyes, preservatives, antioxidants, dyes, thickeners, pH adjusters, perfumes, cooling-sensation agents, antiperspirants, disinfectants, and skin activators. Specifically, a desired cosmetic can be produced in the usual manner using any one or more of the components listed below. The amounts of these components incorporated are not particularly limited as long as they do not interfere with the effects of the present invention.

The oil is not particularly limited, and examples thereof may include avocado oil, camellia oil, turtle oil, macadamia nut oil, corn oil, mink oil, olive oil, rapeseed oil, egg-yolk oil, sesame oil, persic oil, wheat germ oil, sasanqua oil, castor oil, linseed oil, safflower oil, cottonseed oil, perilla oil, soybean oil, arachis oil, tea seed oil, kaya oil, rice bran oil, Chinese tung oil, Japanese tung oil, jojoba oil, germ oil, triglycerol, glycerol trioctanoate, glycerol triisopalmitate, cacao butter, coconut oil, horse fat, hydrogenated coconut oil, palm oil, beef tallow, mutton tallow, hydrogenated beef tallow, palm kernel oil, lard, beef bone fat, Japan wax kernel oil, hydrogenated oil, neatsfoot oil, Japan wax, hydrogenated castor oil, beeswax, candelilla wax, cotton wax, carnauba wax, bayberry wax, insect wax, spermaceti wax, montan wax, bran wax, lanolin, kapok wax, lanolin acetate, liquid lanolin, sugarcane wax, isopropyl lanolate, hexyl laurate, reduced lanolin, jojoba wax, hard lanolin, shellac wax, POE lanolin alcohol ether, POE lanolin alcohol acetate, POE cholesterol ether, polyethylene glycol lanolate, POE hydrogenated lanolin alcohol ether, liquid paraffin, ozokerite, pristane, paraffin, ceresin, squalene, Vaseline, and microcrystalline wax.

The lipophilic nonionic surfactant is not particularly limited, and examples thereof may include sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate; glycerin polyglycerin fatty acids such as glycerol mono-cottonseed oil fatty acid, glycerol monoerucate, glycerol sesquioleate, glycerol monostearate, α,α'- glycerol oleate pyroglutamate, and glycerol monostearate malate; propylene glycol fatty acid esters such as propylene glycol monostearate; hydrogenated castor oil derivatives; and glycerol alkyl ethers.

The hydrophilic nonionic surfactant is not particularly limited, and examples thereof may include POE sorbitan fatty acid esters such as POE sorbitan monooleate, POE sorbitan monostearate and POE sorbitan tetraoleate; POE sorbitol fatty acid esters such as POE sorbitol monolaurate, POE sorbitol monooleate, POE sorbitol pentaoleate and POE sorbitol monostearate; POE glycerin fatty acid esters such as POE glycerin monostearate, POE glycerin monoisostearate and POE glycerin triisostearate; POE fatty acid esters such as POE monooleate, POE distearate, POE monodioleate and ethylene glycol distearate; POE alkyl ethers such as POE lauryl ether, POE oleyl ether, POE stearyl ether, POE behenyl ether, POE 2-octyldodecyl ether and POE cholestanol ether; POE alkyl phenyl ethers such as POE octyl phenyl ether, POE nonyl phenyl ether and POE dinonyl phenyl ether; Pluaronic types such as Pluronic; POE/POP alkyl ethers such as POE/POP cetyl ether, POE/POP 2-decyltetradecyl ether, POE/POP monobutyl ether, POE/POP hydrogenated lanolin and POE/POP glycerin ether; tetra-POE/tetra-POP ethylenediamine condensation products such as Tetronic; POE castor oil hydrogenated castor oil derivatives such as POE castor oil, POE hydrogenated castor oil, POE hydrogenated castor oil monoisostearate, POE hydrogenated castor oil triisostearate, POE hydrogenated castor oil monopyroglutamic acid monoisostearic acid diester and POE hydrogenated castor oil maleic acid; POE beeswax/lanolin derivatives such as POE sorbitol beeswax; alkanolamides such as coconut oil fatty acid diethanolamide, lauric acid monoethanolamide and fatty acid isopropanol amide; POE propylene glycol fatty acid esters; POE alkylamines; POE fatty acid amides; sucrose fatty acid esters; POE nonylphenyl formaldehyde condensation products; alkyl ethoxy dimethylamine oxides; and trioleyl phosphoric acid.

Examples of other surfactants include anionic surfactants such as fatty acid soaps, higher-alkyl sulfuric ester salts, POE triethanolamine lauryl sulfate, and alkyl ether sulfuric ester salts; cationic surfactants such as alkyl trimethylammonium salts, alkyl pyridinium salts, alkyl quaternary ammonium salts, alkyl dimethylbenzyl ammonium salts, POE alkylamines, alkylamine salts, and polyamine fatty acid derivatives; and amphoteric surfactants such as imidazoline amphoteric surfactants and betaine surfactants. They may be incorporated within the bounds of not causing any problems with stability and skin irritation.

The moisturizer is not particularly limited, and examples thereof may include xylitol, sorbitol, maltitol, chondroitin sulfate, hyaluronic acid, mucoitinsulfuric acid, caronic acid, atelocollagen, cholesteryl-12-hydroxystearate, sodium lactate, bile salts, dl-pyrrolidone carboxylate, short-chain soluble collagens, diglycerol (EO) PO adducts, Rosa roxburghii extract, yarrow extract, and melilot extract.

The higher alcohol is not particularly limited, and examples thereof may include linear alcohols such as lauryl alcohol, cetyl alcohol, stearyl alcohol, behenyl alcohol, myristyl alcohol, oleyl alcohol, and cetostearyl alcohol; and branched alcohols such as monostearyl glycerol ether (batyl alcohol), 2-decyltetradecynol, lanolin alcohol, cholesterol, phytosterol, hexyldodecanol, isostearyl alcohol, and octyldodecanol.

The sequestrant is not particularly limited, and examples thereof may include 1-hydroxyethane-1,1-diphosphonic acid, 1-hydroxyethane-1,1-diphosphonic acid tetrasodium salt, sodium citrate, sodium polyphosphate, sodium metaphosphate, gluconic acid, phosphoric acid, citric acid, ascorbic acid, succinic acid, and edetic acid.

The natural water-soluble polymer is not particularly limited, and examples thereof may include plant-derived polymers such as gum arabic, tragacanth gum, galactan, guar gum, carob gum, karaya gum, carrageenan, pectin, agar, quince seed (quince), algal colloid (algal extract), starch (rice, corn, potato, wheat), and glycyrrhizinic acid; microorganism-derived polymers such as xanthan gum, dextran, succinoglucan, and pullulan; and animal-derived polymers such as collagen, casein, albumin, and gelatin.

The semisynthetic water-soluble polymer is not particularly limited, and examples thereof may include starch polymers such as carboxymethyl starch and methyl hydroxypropyl starch; cellulose polymers such as methyl cellulose, nitro cellulose, ethyl cellulose, methyl hydroxypropyl cellulose, hydroxyethyl cellulose, cellulose sodium sulfate, hydroxypropyl cellulose, sodium carboxymethylcellulose (CMC), crystalline cellulose, and cellulose powder; and alginate polymers such as sodium alginate and propylene glycol alginate.

The synthetic water-soluble polymer is not particularly limited, and examples thereof may include vinyl polymers such as polyvinyl alcohol, polyvinyl methyl ether, and polyvinyl pyrrolidone; polyoxyethylene polymers such as polyethylene glycol 20,000, polyethylene glycol 40,000, and polyethylene glycol 60,000; copolymers such as polyoxyethylene-polyoxypropylene copolymers; acrylic polymers such as sodium polyacrylate, polyethylacrylate, and polyacrylamide; polyethyleneimine; and cationic polymers.

The inorganic water-soluble polymer is not particularly limited, and examples thereof may include bentonite, magnesium aluminum silicate (Veegum), laponite, hectorite, and silicic anhydride.

The ultraviolet blocking agent is not particularly limited, and examples thereof may include benzoic acid-based ultraviolet blocking agents such as paraaminobenzoic acid (hereinafter, abbreviated as PABA), PABA monoglycerin ester, N,N-dipropoxy PABA ethyl ester, N,N-diethoxy PABA ethyl ester, N,N-dimethyl PABA ethyl ester and N,N-dimethyl PABA butyl ester; anthranilic acid-based ultraviolet blocking agents such as homomenthyl-N-acetyl anthranilate; salicylic acid-based ultraviolet blocking agents such as amyl salicylate, menthyl salicylate, homomenthyl salicylate, octyl salicylate, phenyl salicylate, benzyl salicylate and p-isopropanol phenyl salicylate; cinnamic acid-based ultraviolet blocking agents such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate and glycerylmono-2-ethylhexanoyl-diparamethoxy cinnamate; benzophenone-based ultraviolet blocking agents such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone and 4-hydroxy-3-carboxybenzophenone; 3-(4'-methylbenzylidene)-d,l-camphor, 3-benzylidene-d,l-camphor, urocanic acid, urocanic acid ethyl ester, 2-phenyl-5-methylbenzoxazole, 2,2'-hydroxy-5-methylphenyl benzotrialzole, 2-(2'-hydroxy-5'-t-octylphenyl)benzotriazole, 2-(2'-hydroxy-5'-methylphenyl benzotriazole, dibenzalazine, dianisoylmethane, 4-methoxy-4'-t-butyldibenzoylmethane and 5-(3,3-dimethyl-2-norbornylidene)-3-pentane-2-one.

Other chemical components are not particularly limited, and examples thereof may include vitamins such as vitamin A oil, retinol, retinol palmitate, inositol, pyridoxine hydrochloride, benzyl nicotinate, nicotinamide, DL-α-tocopherol nicotinate, magnesium ascorbyl phosphate, 2-O-α-D-glucopyranosyl-L-ascorbic acid, vitamin D2 (ergocalciferol), DL-α-tocopherol, DL-α-tocopherol acetate, pantothenic acid, and biotin; hormones such as estradiol and ethynyl estradiol; amino acids such as arginine, aspartic acid, cystine, cysteine, methionine, serine, leucine, and tryptophan; anti-inflammatory agents such as allantoin and azulene; whitening agents such as arbutin; astringents such as tannic acid; refrigerants such as L-menthol and camphor, sulfur, lysozyme chloride, and pyridoxine chloride.

Various kinds of extracts are not particularly limited, and examples thereof may include Houttuynia cordata extract, Phellodendron bark extract, melilot extract, dead nettle extract, licorice extract, peony root extract, soapwort extract, luffa extract, cinchona extract, strawberry geranium extract, sophora root extract, nuphar extract, fennel extract, primrose extract, rose extract, rehmannia root extract, lemon extract, lithospermum root extract, aloe extract, calamus root extract, eucalyptus extract, field horsetail extract, sage extract, thyme extract, tea extract, seaweed extract, cucumber extract, clove extract, bramble extract, lemon balm extract, carrot extract, horse chestnut extract, peach extract, peach leaf extract, mulberry extract, knapweed extract, hamamelis extract, placenta extract, thymic extract, silk extract, and licorice extract.

Examples of the various kinds of powders may include bright coloring pigments such as red oxide, yellow iron oxide, black iron oxide, mica titanium, iron oxide-coated mica titanium and titanium oxide-coated glass flakes, inorganic powders such as those of mica, talc, kaolin, sericite, titanium dioxide and silica, and organic powders such as polyethylene powder, nylon powder, crosslinked polystyrene, cellulose powder and silicone powder. Preferably, part or all of the powder component is subjected to a known hydrophobization treatment with a substance such as a silicone, a fluorine compound, a metallic soap, an oily agent or an acyl glutamic acid salt for improvement of sensory characteristics and improvement of makeup retainability.

### EXAMPLES

Hereinafter, the present disclosure will be explained with reference to examples. However, the present disclosure is not limited to these examples.

### (Production method of inorganic particle composed of a compound represented by CaAl₁₂O₁₉:Mn⁴⁺)

Calcium carbonate (manufactured by Sakai Chemical Industry Co., Ltd., CWS-20, 4.97 g), manganese carbonate (manufactured by Chuo Denki Kogyo Co., Ltd., 0.06 g), aluminum oxide (manufactured by Iwatani Chemical Industry Co., Ltd., RG-40, 32.0 g), and calcium fluoride (first class grade chemical manufactured by Wako Pure Chemical Industries, Ltd., 0.18 g) and magnesium fluoride (special grade chemical manufactured by Wako Pure Chemical Industries, Ltd., 0.15 g) as flux components were weighed and put in water. Then, the mixture was stirred sufficiently using a planetary ball mill at 250 rpm for 30 minutes. The mixed slurry was dried by evaporation at 130°C and the obtained solid matter was crushed by using a mortar to obtain calcinating precursor powder. Next, the calcinating precursor 15 g was put in an alumina crucible and heated to 1600°C at 200°C/hour in the atmosphere. After keeping the temperature for 3 hours, the matter was cooled to room temperature at 200°C/hour.

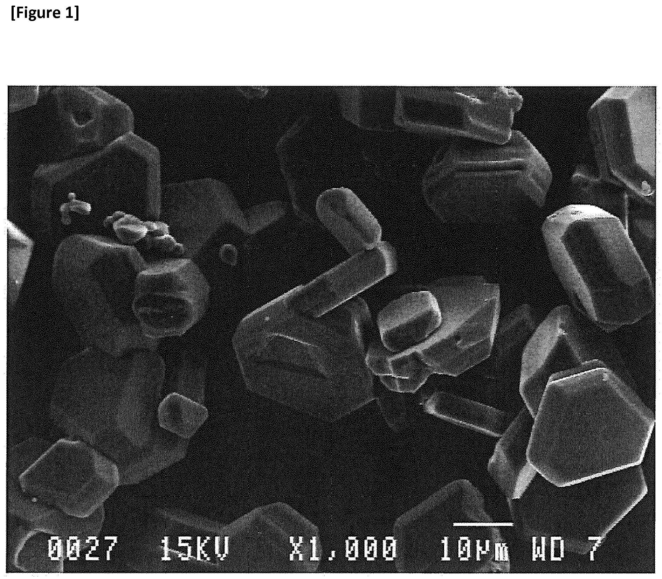

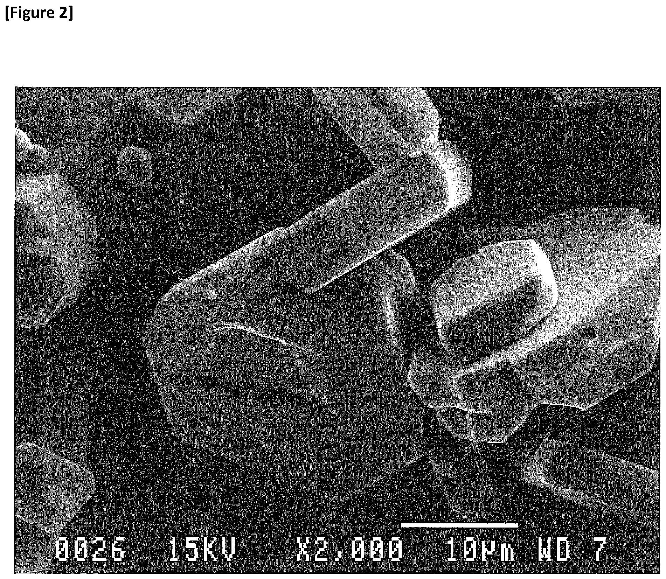

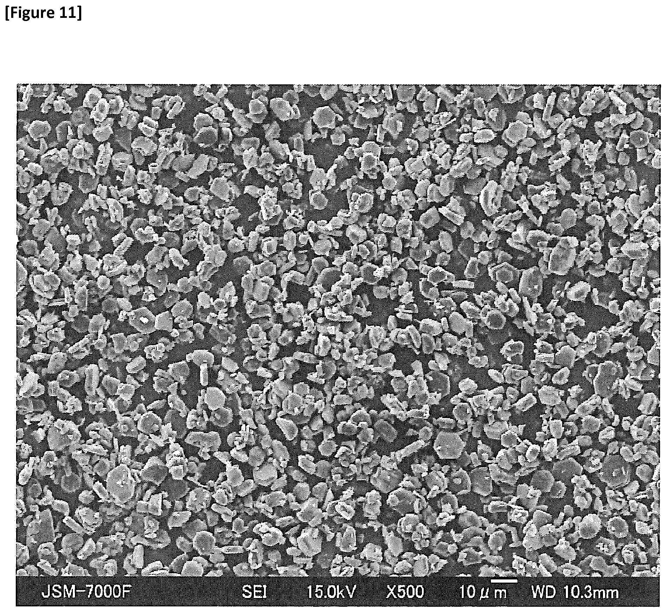

The calcinated matter thus obtained was crushed by using a mortar to obtain compound P1. The obtained electron microscope photographs of P1 are shown in Figs. 1 and 2, and the measurement result of XRD is shown in FIG.11. When P1 is expressed by a general formula, CaₓAl_{y}O_{(2x+3y+4z)/2}:Mn⁴⁺_{z}, X=0.99, Y=11.96, and Z=0.01.

The specific surface area measured by using Macsorb Model HM-1220 manufactured by Mountech Co., Ltd. at the deaeration temperature of 230°C and for the deaeration time of 35 minutes and the particle size distribution measured by using a laser diffraction particle size distribution analyzer (MT-3300 manufactured by NIKKISO CO.,LTD) are shown in table 7. The obtained sample was used and a powder foundation was prepared according to the composition shown in table 1. The materials used for the foundation were as stated below and materials other than P1 were cosmetic grades.
Mica: Y-2300X (manufactured by Yamaguchi Mica Co., Ltd.) Sericite: FSE (manufactured by Sanshin Mining Ind. Co., Ltd.) Plate-shaped barium sulfate: plate-shaped barium sulfate-H (manufactured by Sakai Chemical Industry Co., Ltd.) Spherical silicone: KSP-105 (manufactured by Shin-Etsu Chemical Co., Ltd.)
Titanium oxide: R-3LD (manufactured by Sakai Chemical Industry Co., Ltd.)
Iron oxide (yellow): Yellow iron oxide (manufactured by PINOA Co., Ltd.)
Iron oxide (red): Red iron oxide (manufactured by PINOA Co., Ltd.)
Metal soap: JPM-100 (manufactured by Sakai Chemical Industry Co., Ltd.)
Oil: KF96 (manufactured by Shin-Etsu Chemical Co., Ltd.)

**Table 1**

| | |
|---|---|
| Mica | 24.30 |
| Sericite | 29.16 |
| Plate-shaped barium sulfate | 11.70 |
| Spherical silicone | 6.30 |
| Titanium oxide | 7.20 |
| Iron oxide (yellow) | 1.08 |
| Iron oxide (red) | 0.36 |
| Metal soap | 0.90 |
| Oil | 9.00 |
| P1 | 10.00 |
| Total | 100.00 |

These materials were weighed according to the composition and mixed while stirring by a coffee mill for one minute and 30 seconds. The obtained powder mixture 0.8 g was weighed in a metal mold with a diameter of 20 mmΦ and pressed using a press machine under a pressure of 200 kgf/cm² for 30 seconds to obtain powder foundation F1.

### Example 2

Calcium carbonate (manufactured by Sakai Chemical Industry Co., Ltd., CWS-20, 4.97 g), manganese carbonate (manufactured by Chuo Denki Kogyo Co., Ltd., 0.06 g), aluminum oxide (manufactured by Iwatani Chemical Industry Co., Ltd., RG-40, 32.0 g), and calcium fluoride (first class grade chemical manufactured by Wako Pure Chemical Industries, Ltd., 0.18 g) and magnesium fluoride (special grade chemical manufactured by Wako Pure Chemical Industries, Ltd., 0.15 g) as flux components were weighed and put in water. Then, the mixtures was stirred sufficiently using a planetary ball mill at 250 rpm for 30 minutes. The mixed slurry was dried by evaporation at 130°C and the obtained solid matter was crushed by using a mortar to obtain calcinating precursor powder. Next, the calcinating precursor 15 g was put in an alumina crucible and heated to 1600°C at 200°C/hour in the atmosphere. After keeping the temperature for 3 hours, the matter was cooled to room temperature at 200°C/hour.

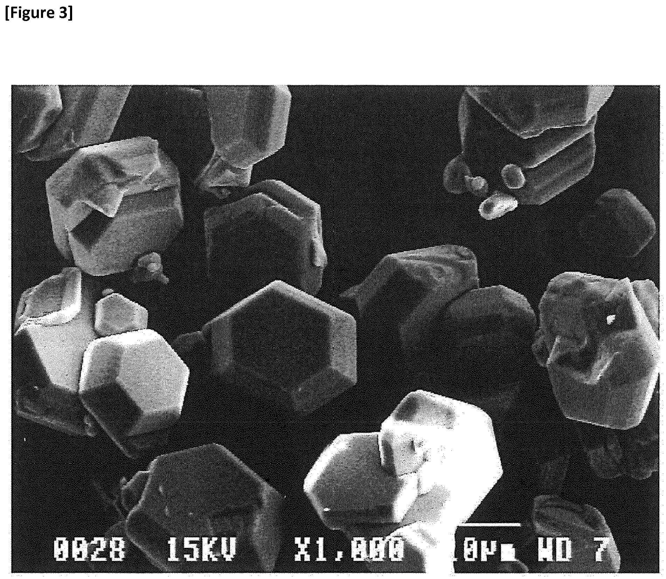

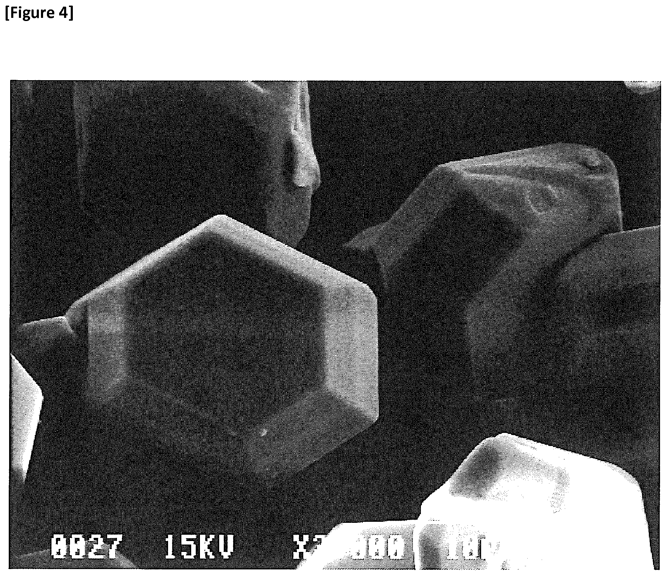

The calcinated matter thus obtained was pulverized in water by using a planetary ball mill and the pulverized slurry was washed with diluted hydrochloric acid. After washing, the slurry was washed with a great amount of water, filtrated and dried. After drying at 130°C, the obtained solid matter was crushed. Next, the crushed matter 10 g was put in an alumina crucible and heated to 1600°C at 200°C/hour in the atmosphere. After keeping the temperature for 3 hours, the matter was cooled to room temperature at 200°C/hour.
The calcinated matter thus obtained was crushed by using a mortar to obtain compound P2. The obtained electron microscope photographs of P2 are shown in Figs. 3 and 4. When P2 is expressed by a general formula, CaₓAl_{y}O_{(2x+3y+4z)/2}:Mn⁴⁺_{z}, X=0.99, Y=11.96, and Z=0.01.

The specific surface area and the particle size measured by following the same procedure of Example 1 are shown in table 7. The obtained sample was used and a powder foundation was prepared according to the composition shown in table 2. The materials used for the foundation other than P2 were cosmetic grades.

**Table 2**

| | |
|---|---|
| Mica | 24.30 |
| Sericite | 29.16 |
| Plate-shaped barium sulfate | 11.70 |
| Spherical silicone | 6.30 |
| Titanium oxide | 7.20 |
| Iron oxide (yellow) | 1.08 |
| Iron oxide (red) | 0.36 |
| Metal soap | 0.90 |
| Oil | 9.00 |
| P2 | 10.00 |
| Total | 100.00 |

These materials were weighed according to the composition and mixed while stirring by a coffee mill for one minute and 30 seconds. The obtained powder mixture 0.8 g was weighed in a metal mold with a diameter of 20 mmΦ and pressed using a press machine under a pressure of 200 kgf/cm² for 30 seconds to obtain powder foundation F2.

### Example 3

Calcium carbonate (manufactured by Sakai Chemical Industry Co., Ltd., CWS-20, 4.87 g), manganese carbonate (manufactured by Chuo Denki Kogyo Co., Ltd., 0.19 g), aluminum oxide (manufactured by Iwatani Chemical Industry Co., Ltd., RG-40, 32.0 g), and calcium fluoride (first class grade chemical manufactured by Wako Pure Chemical Industries, Ltd., 0.18 g) and magnesium fluoride (special grade chemical manufactured by Wako Pure Chemical Industries, Ltd., 0.15 g) as flux components were weighed and put in water. Then, the mixtures was stirred sufficiently using a planetary ball mill at 250 rpm for 30 minutes. The mixed slurry was dried by evaporation at 130°C and the obtained solid matter was crushed by using a mortar to obtain calcinating precursor powder. Next, the calcinating precursor 15 g was put in an alumina crucible and heated to 1600°C at 200°C/hour in the atmosphere. After keeping the temperature for 3 hours, the matter was cooled to room temperature at 200°C/hour.

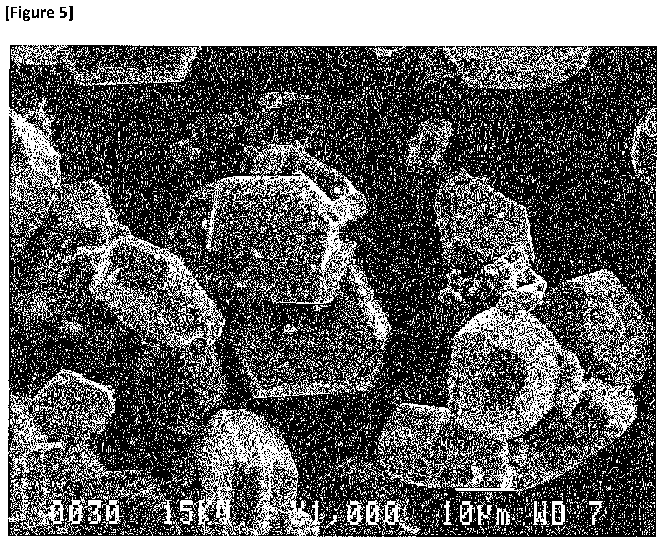

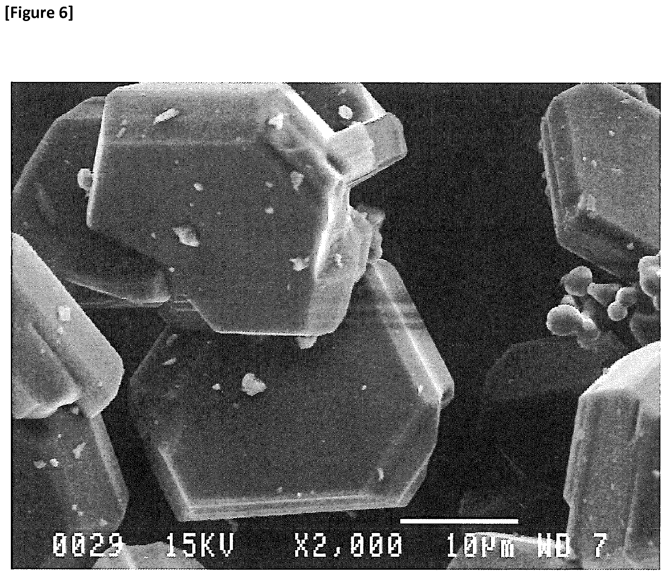

The calcinated matter thus obtained was crushed by using a mortar to obtain compound P3. The obtained electron microscope photographs of P3 are shown in Figs. 5 and 6. When P3 is expressed by a general formula, CaₓAl_{y}O_{(2x+3y+4z)/2}:Mn⁴⁺_{z}, X=0.97, Y=11.96, and Z=0.03.

The specific surface area and the particle size measured by following the same procedure of Example 1 are shown in table 7. The obtained sample was used and a powder foundation was prepared according to the composition shown in table 3. The materials used for the foundation other than P3 were cosmetic grades.

**Table 3**

| | |
|---|---|
| Mica | 24.30 |
| Sericite | 29.16 |
| Plate-shaped barium sulfate | 11.70 |
| Spherical silicone | 6.30 |
| Titanium oxide | 7.20 |
| Iron oxide (yellow) | 1.08 |
| Iron oxide (red) | 0.36 |
| Metal soap | 0.90 |
| Oil | 9.00 |
| P3 | 10.00 |
| Total | 100.00 |

These materials were weighed according to the composition and mixed while stirring by a coffee mill for one minute and 30 seconds. The obtained powder mixture 0.8 g was weighed in a metal mold with a diameter of 20 mmΦ and pressed using a press machine under a pressure of 200 kgf/cm² for 30 seconds to obtain powder foundation F3.

### Example 4

Calcium carbonate (manufactured by Sakai Chemical Industry Co., Ltd., CWS-20, 4.74 g), manganese carbonate (manufactured by Chuo Denki Kogyo Co. , Ltd., 0.06 g), aluminum oxide (manufactured by Iwatani Chemical Industry Co., Ltd., RG-40, 32.1 g), and calcium fluoride (first class grade chemical manufactured by Wako Pure Chemical Industries, Ltd., 0.37 g) as flux components were weighed and put in water. Then, the mixtures was stirred sufficiently using a planetary ball mill at 250 rpm for 30 minutes. The mixed slurry was dried by evaporation at 130°C and the obtained solid matter was crushed by using a mortar to obtain calcinating precursor powder. Next, the calcinating precursor 15 g was put in an alumina crucible and heated to 1600°C at 200°C/hour in the atmosphere. After keeping the temperature for 3 hours, the matter was cooled to room temperature at 200°C/hour.

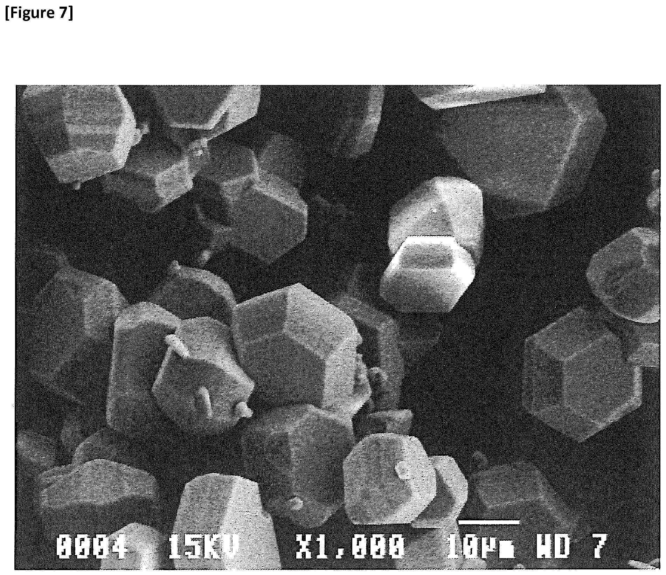

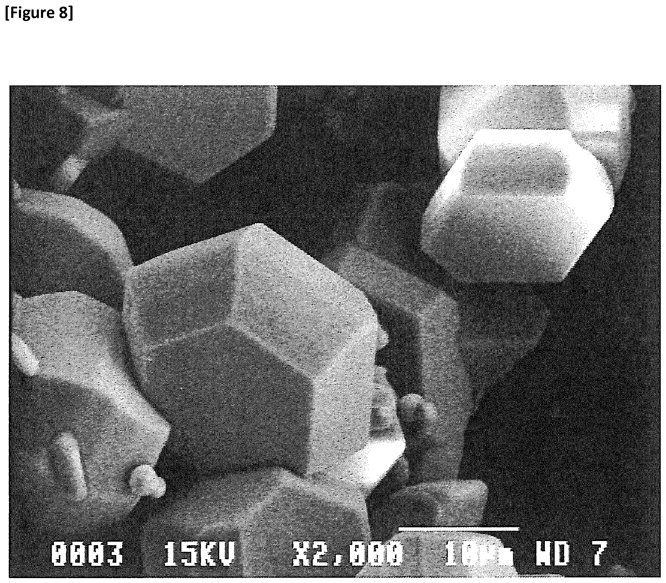

The calcinated matter thus obtained was crushed by using a mortar to obtain compound P4. The obtained electron microscope photographs of P4 are shown in Figs. 7 and 8. When P1 is expressed by a general formula, CaₓAl_{y}O_{(2x+3y+4z)/2}:Mn⁴⁺_{z}, X=0.99, Y=12.00, and Z=0.01.

The specific surface area and the particle size measured by following the same procedure of Example 1 are shown in table 7. The obtained sample was used and a powder foundation was prepared according to the composition shown in table 4. The materials used for the foundation other than P4 were cosmetic grades.

**Table 4**

| | |
|---|---|
| Mica | 24.30 |
| Sericite | 29.16 |
| Plate-shaped barium sulfate | 11.70 |
| Spherical silicone | 6.30 |
| Titanium oxide | 7.20 |
| Iron oxide (yellow) | 1.08 |
| Iron oxide (red) | 0.36 |
| Metal soap | 0.90 |
| Oil | 9.00 |
| P4 | 10.00 |
| Total | 100.00 |

These materials were weighed according to the composition and mixed while stirring by a coffee mill for one minute and 30 seconds. The obtained powder mixture 0.8 g was weighed in a metal mold with a diameter of 20 mmΦ and pressed using a press machine under a pressure of 200 kgf/cm² for 30 seconds to obtain powder foundation F4.

### Example 5

Calcium carbonate (manufactured by Sakai Chemical Industry Co., Ltd., CWS-20, 5.21 g), manganese carbonate (manufactured by Chuo Denki Kogyo Co., Ltd., 0.06 g), aluminum oxide (manufactured by Iwatani Chemical Industry Co., Ltd., RG-40, 31.9 g), and magnesium fluoride (special grade chemical manufactured by Wako Pure Chemical Industries, Ltd., 0.29 g) as flux components were weighed and put in water. Then, the mixtures was stirred sufficiently using a planetary ball mill at 250 rpm for 30 minutes. The mixed slurry was dried by evaporation at 130°C and the obtained solid matter was crushed by using a mortar to obtain calcinating precursor powder. Next, the calcinating precursor 15 g was put in an alumina crucible and heated to 1600°C at 200°C/hour in the atmosphere. After keeping the temperature for 3 hours, the matter was cooled to room temperature at 200°C/hour.

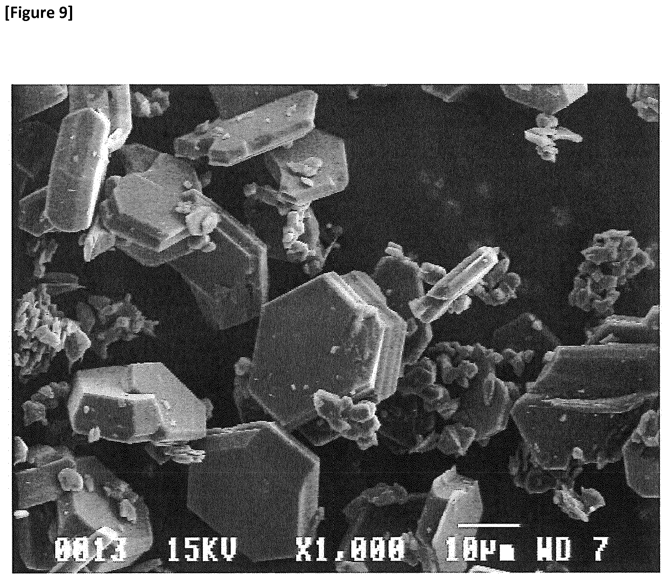

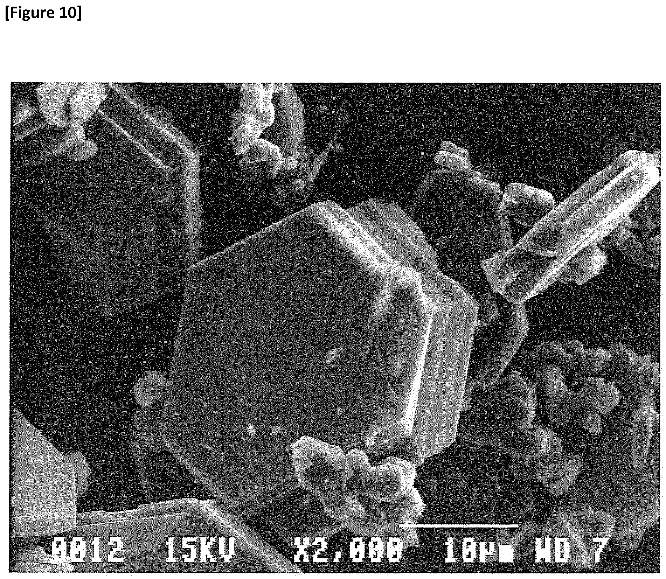

The calcinated matter thus obtained was crushed by using a mortar to obtain compound P5. The obtained electron microscope photographs of P5 are shown in Figs. 9 and 10. When P5 is expressed by a general formula, CaₓAl_{y}O_{(2x+3y+4z)/2}:Mn⁴⁺_{z}, X=0.99, Y=11.91, and Z=0.01. The specific surface area and the particle size measured by following the same procedure of Example 1 are shown in table 7. The obtained sample was used and a powder foundation was prepared according to the composition shown in table 5. The materials used for the foundation other than P5 were cosmetic grades.

**Table 5**

| | |
|---|---|
| Mica | 24.30 |
| Sericite | 29.16 |
| Plate-shaped barium sulfate | 11.70 |
| Spherical silicone | 6.30 |
| Titanium oxide | 7.20 |
| Iron oxide (yellow) | 1.08 |
| Iron oxide (red) | 0.36 |
| Metal soap | 0.90 |
| Oil | 9.00 |
| P5 | 10.00 |
| Total | 100.00 |

These materials were weighed according to the composition and mixed while stirring by a coffee mill for one minute and 30 seconds. The obtained powder mixture 0.8 g was weighed in a metal mold with a diameter of 20 mmΦ and pressed using a press machine under a pressure of 200 kgf/cm² for 30 seconds to obtain powder foundation F5.

### Example 6

Calcium carbonate (manufactured by Sakai Chemical Industry Co., Ltd., CWS-20, 5.21 g), manganese carbonate (manufactured by Chuo Denki Kogyo Co., Ltd., 0.06 g), aluminum oxide (manufactured by Iwatani Chemical Industry Co., Ltd., RG-40, 31.9 g), and boric acid (special grade chemical manufactured by Wako Pure Chemical Industries, Ltd., 0.29 g) as flux components were weighed and put in water. Then, the mixtures was stirred sufficiently using a planetary ball mill at 250 rpm for 30 minutes. The mixed slurry was dried by evaporation at 130°C and the obtained solid matter was crushed by using a mortar to obtain calcinating precursor powder. Next, the calcinating precursor 15 g was put in an alumina crucible and heated to 1300°C at 200°C/hour in the atmosphere. After keeping the temperature for 3 hours, the matter was cooled to room temperature at 200°C/hour.

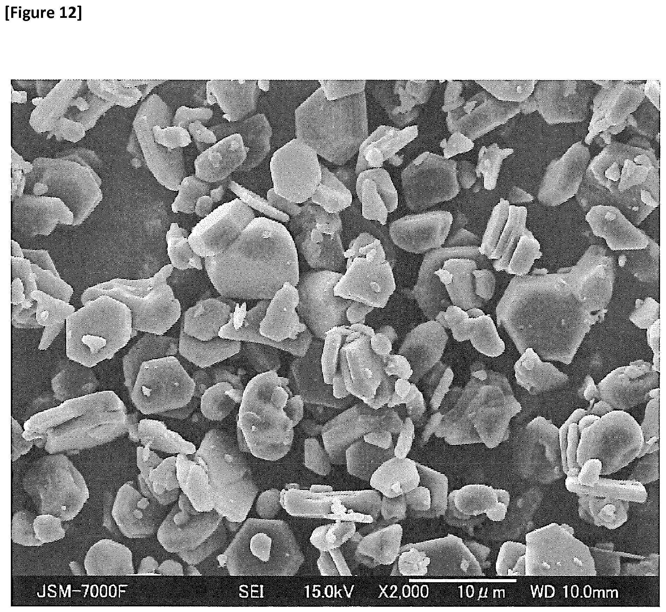

The calcinated matter thus obtained was crushed by using a mortar to obtain compound P6. The obtained electron microscope photographs of P6 are shown in Figs. 11 and 12. When P6 is expressed by a general formula, CaₓAl_{y}O_{(2x+3y+4z)/2}:Mn⁴⁺_{z}, X=0.99, Y=11.91, and Z=0.01. The specific surface area and the particle size measured by following the same procedure of Example 1 are shown in table 7. The obtained sample was used and a powder foundation was prepared according to the composition shown in table 5. The materials used for the foundation other than P6 were cosmetic grades.

**Table 6**

| | |
|---|---|
| Mica | 24.30 |
| Sericite | 29.16 |
| Plate-shaped barium sulfate | 11.70 |
| Spherical silicone | 6.30 |
| Titanium oxide | 7.20 |
| Iron oxide (yellow) | 1.08 |
| Iron oxide (red) | 0.36 |
| Metal soap | 0.90 |
| Oil | 9.00 |
| P6 | 10.00 |
| Total | 100.00 |

These materials were weighed according to the composition and mixed while stirring by a coffee mill for one minute and 30 seconds. The obtained powder mixture 0.8 g was weighed in a metal mold with a diameter of 20 mmΦ and pressed using a press machine under a pressure of 200 kgf/cm² for 30 seconds to obtain powder foundation F6.

### Comparative example 1 (Preparation of fluorescent substance free foundation)

A powder foundation was prepared the composition shown in table 6. The all materials used for the foundation were cosmetic grades.

**Table 7**

| | |
|---|---|
| Mica | 27.00 |
| Sericite | 32.40 |
| Plate-shaped barium sulfate | 13.00 |
| Spherical silicone | 7.00 |
| Titanium oxide | 8.00 |
| Iron oxide (yellow) | 1.20 |
| Iron oxide (red) | 0.40 |
| Metal soap | 1.00 |
| Oil | 10.00 |
| Total | 100.00 |

These materials were weighed according to the composition and mixed while stirring by a coffee mill for one minute and 30 seconds. The obtained powder mixture 0.8 g was weighed in a metal mold with a diameter of 20 mmΦ and pressed using a press machine under a pressure of 200 kgf/cm² for 30 seconds to obtain powder foundation F7.

### Evaluation example 1 (Evaluation as fluorescent substance)

The fluorescent substance samples P1 to P6 obtained in examples were evaluated.

### [Measurement of luminance Y]

The emission spectra was measured by using FP-6500 manufactured by JASCO Corporation where the excitation wavelength was set at 450 nm. ISF-513 type was used as a fluorescent integral sphere, and the value of voltage of photomultiplier tube was set at 400.

### • Relative luminance

The Y value of P1 was set to 100, and the relative luminance of P1 to P6 respectively was calculated. The results are shown in table 7.

### [Shape of fluorescent substance particle]

The SEM photographs of P1 to P6 with 1000 and 2000 magnifications were taken by using a SEM (840F manufactured by JEOL Ltd.) for confirming the shape of the fluorescent substance particles. The results are shown in Figs. 1 to 10. From Figs. 1 to 10, it is clear that the inorganic particles obtained in examples are hexagonal-plate shaped.

### [Identification of fluorescent substance]

The measurement by XRD (RINT-TTRIII manufactured by Rigaku Corporation, x-rays = CuKα, λ1.5406Å, 50kV, 300mA) was performed for identification of the product P1 obtained in example 1. The measurement result matched PDF card #01-084-1613 (RDB), and it was confirmed that the composition of P1 was almost the same as CaAl₁₂O₁₉:Mn⁴⁺ from the result of Fig. 11. The result is shown in Fig. 11.

### Evaluation example 2 (Evaluation as cosmetic)

The foundations of F1 to F7 obtained in examples 1 to 6 and comparative example 1 were applied by ten panelists under the condition that the numbers of foundations were not noticed by the panelists. Then, the color was tested with the irradiation of black light at 365 nm. The results of evaluation into five levels of reddish tint are shown in table 8.

**Table 8**

| | P1 | P2 | P3 | P4 | P5 | P6 |
|---|---|---|---|---|---|---|
| Luminance Y | 0.6 | 0.7 | 0.4 | 0.2 | 0.6 | 0.6 |
| Relative luminance Y (Relative to P1/%) | 100.00 | 116.7 | 66.7 | 33.3 | 100.0 | 100.0 |
| Specific surface area (m²/g) | 0.07 | 0.08 | 0.1 | 0.09 | 0.26 | 0.60 |
| Particle size D10 (µm) | 25.16 | 24.22 | 21.48 | 25.56 | 14.81 | 4.9 |
| Particle size D50 (µm) | 43.61 | 36.76 | 40.18 | 49.04 | 31.79 | 7.5 |
| Particle size D90 (µm) | 72.95 | 56.59 | 72.19 | 92.06 | 104.1 | 10.6 |

**Table 9**

| | F1 | F2 | F3 | F4 | F5 | F6 | F7 |
|---|---|---|---|---|---|---|---|
| There is a reddish tint | 5 | 5 | 3 | 3 | 4 | 5 | 1 |

| | | | | | | | |
|---|---|---|---|---|---|---|---|
| 5: More than 8 or all panelists recognized reddish tint. 4: 5 to 7 panelists recognized reddish tint. 3: 2 to 4 panelists recognized reddish tint. 2: one panelist recognized reddish tint. 1: No panelist recognized reddish tint. | | | | | | | |

From these results, it is clear that the powder of the present disclosure can improve the optical functions.

### INDUSTRIAL APPLICABILITY

The cosmetic of the present disclosure may have excellent effects in covering color of the skin and imparting transparency to the skin so that it can be used suitably as makeup cosmetics including a foundation.

The cosmetic of the present disclosure can develop the effect especially outside under natural light because the reddish tint becomes strong when an ultraviolet ray at 365 nm in natural light (sunlight) is irradiated. In addition, the cosmetic can develop the effect even indoors because emission is generated in the visible region.

## Claims

1. A cosmetic containing inorganic particles consisting of a compound represented by a general formula:
CaₓAl_{y}O_{(2x+3y+4z)/2}:Mn⁴⁺_{z}
wherein 0.1<x<1.05, 11.9<y≦12, and 0.0005<z<0.1.

2. The cosmetic according to claim 2 having the inorganic particle content of 0.1 to 100 wt% relative to the total amount of the cosmetic.

3. The cosmetic according to claim 1 or 2, wherein the inorganic particles have a hexagonal plate shape.

4. The cosmetic according to any one of claims 1 to 4, the inorganic particles have an average particle diameter of 5 to 100 µm.

5. The cosmetic according to any one of claims 1 to 4, the inorganic particles have a luminance Y of 0.1 or more when a light having an excitation light wavelength of 450 nm is irradiated.
